# NEUE EUROPÄISCHE PATENTSCHRIFT

(11) **EP 2 252 350 B2**
(45) Veröffentlichungstag und Bekanntmachung des Hinweises auf die Entscheidung über den Einspruch: **28.05.2025**
(45) Hinweis auf die Patenterteilung: 20.04.2016
(21) Anmeldenummer: 09710102.6
(22) Anmeldetag: 04.02.2009
(51) Int. Cl.: A61M 5/20, A61M 5/24, A61M 5/315, A61M 5/50

(54) **VERABREICHUNGSVORRICHTUNG MIT BLOCKIERBAREM BETÄTIGUNGSELEMENT**
ADMINISTERING APPARATUS COMPRISING A BLOCKABLE ACTUATION ELEMENT
SYSTÈME D'ADMINISTRATION DE PRODUIT À ÉLÉMENT D'ACTIONNEMENT POUVANT ÊTRE BLOQUÉ

(30) Priorität: 11.02.2008 CH 189082008
(43) Veröffentlichungstag der Anmeldung: 24.11.2010
(73) Patentinhaber: Ypsomed AG, 3401 Burgdorf (CH)
(72) Erfinder: HIRSCHEL, Jürg, CH-5000 Aarau (CH); KÄNEL-JOST, Céline, CH-8032 Zürich (CH); MOSER, Ulrich, CH-3412 Heimiswil (CH); DRUNK, Annette, CH-3007 Bern (CH); TSCHIRREN, Markus, CH-3422 Kirchberg (CH)
(74) Vertreter: SSM Sandmair
(86) Internationale Anmeldenummer: PCT/CH2009/000042
(87) Internationale Veröffentlichungsnummer: WO 2009/100550

(56) Entgegenhaltungen:
- EP-A1- 0 562 671
- WO-A1-2007/051331
- WO-A1-98/20921
- WO-A2-2007/002052
- DE-A1- 102005 007 614
- DE-A1- 102006 017 209
- US-A- 5 569 192
- US-A1- 2007 135 767
- US-B1- 6 572 581
- US-B1- 6 572 581
- US-B2- 6 893 420
- US-B2- 6 893 420

## Beschreibung

### Technisches Gebiet

Die vorliegende Erfindung betrifft eine Vorrichtung zur Verabreichung eines fluiden Produkts, wie z. B. fluide Medikamente, Pharmazeutika oder Kosmetika. Insbesondere betrifft die Erfindung eine Vorrichtung zur Verabreichung eines fluiden Produkts, welches in einer Zweikammerkarpule vor der Anwendung abzumischen ist.

### Stand der Technik

Bei der Behandlung verschiedener Erkrankungen, wie z.B. Diabetes und bei Wachstumsstörungen, werden Injektionsgeräte, so genannte Pens, verwendet, um das Medikament in Form eines fluiden Produkts in das Körpergewebe zu injizieren. Derartige Pens können auch zu pharmazeutischen oder kosmetischen Zwecken verwendet werden. Ein Pen besteht im Wesentlichen aus einer Verabreichungseinrichtung, welche in einem Pengehäuse untergebracht ist, und einer Aufnahmevorrichtung zur Aufnahme des fluiden Produkts, wie z.B. einem Karpulenhalter, der eine Karpule in sich aufnimmt und mit dem Pengehäuse verbunden wird, um die Karpule an die Verabreichungseinrichtung anzuschliessen. Die Verabreichungseinrichtung besteht in der Regel aus einer Mechanik, welche einen Stopfen in der Karpule antreiben kann. An dem der Mechanik gegenüberliegenden Ende des Karpulenhalters wird im Allgemeinen eine Injektionsnadeleinheit angebracht, welche eine Fluidverbindung zu dem fluiden Produkt in der Karpule bildet. Der Injektionspen umfasst einen Auslöseknopf, durch dessen Betätigung die Verabreichungsmechanik aktiviert wird, so dass durch die Injektionsnadel das Medikament aus der Karpule injiziert werden kann. Aus dem Stand der Technik ist bekannt, den Auslöseknopf zu blockieren, abzudecken oder anderweitig gegen ein ungewolltes Auslösen zu Sichern. Die Blockierung oder Sicherung wird kurz vor der Anwendung des Injektionspens gelöst, um die Injektion vornehmen zu können.

In der Praxis finden oftmals Zweikammer-Karpulen Anwendung, die z.B. für den Einsatz von Hormonpräparaten vorgesehen sind. Die Zweikammer-Karpulen weisen eine erste Kammer mit einem lyophilisierten Wirkstoff und eine zweite Kammer mit einem Lösungsmittel auf. Der Wirkstoff wird erst kurz vor der Verabreichung in dem Lösungsmittel gelöst, in dem das Lösungsmittel in die erste Kammer mit dem Wirkstoff geleitet wird. Diese Zweikammer-Karpulen weisen zwei Stopfen auf welche die beiden Kammern von einander trennen. Beim Abmischen des Wirkstoffes werden die beiden Stopfen innerhalb der Karpule derart verschoben, dass das Lösungsmittel über einen Bypass in die Wirkstoffkammer gelangen kann. Vor allem bei der Verwendung derartiger Zweikammer-Karpulenn in einem Injektionspen ist es wichtig, das keine ungewünschte oder vorzeitige Verabreichung eingeleitet wird, da in einem solchen Fall noch keine vollständige Abmischung des Medikaments vorliegen kann.

Aus der WO2007/002052A2 ist ein Autoinjektor mit einem Anzeigesystem bekannt, wobei der gebrauchsbereite Zustand des Autoinjektors anzeigbar ist. Dabei ist ein Auslöseknopf des Autoinjektors aus dem Gehäuse des Autoinjektors bewegbar.

Ferner ist aus der US 6,893,420B2 ist eine Zweikammer-Injektionsvorrichtung bekannt, wobei die Injektion erst nach dem Abmischvorgang auslösbar ist.

Es ist eine Aufgabe der vorliegenden Erfindung, eine Vorrichtung zur Verabreichung eines fluiden Produktes zu schaffen, bei dem ein unbeabsichtigtes oder vorzeitiges Betätigen der Vorrichtung verhindert wird und somit die Sicherheit der Vorrichtung erhöht wird.

Diese Aufgabe wird durch eine Vorrichtung zur Verabreichung eines fluiden Produkts gemäss den Patentansprüchen 1, 6 und 10 erfüllt. Bevorzugte Ausführungsformen einer solchen Vorrichtung gehen aus den Unteransprüchen hervor.

Demnach umfasst die Vorrichtung zur Verabreichung eines fluiden Produkts ein Gehäuse zur Aufnahme einer Verabreichungseinrichtung, ein Betätigungselement zum Betätigen der Verabreichungseinrichtung, eine Aufnahmevorrichtung zur Aufnahme des fluiden Produkts und eine Blockiereinrichtung zum Blockieren des Betätigungselements. Das Gehäuse der Vorrichtung kann hülsenartig ausgebildet sein, so dass die Vorrichtung die Gestalt eines Pens, bzw. Stiftes annimmt. Wesentlich bei der Gestaltung des Gehäuses ist, dass die Verabreichungseinrichtung darin ohne Beeinträchtigung ihrer Funktion untergebracht werden kann. Es ist auch möglich, dass das Gehäuse Funktionselemente der Verabreichungseinrichtung aufweist und somit einen Teil der Verabreichungseinrichtung bildet. Die Verabreichungseinrichtung besteht z.B. aus einer Mechanik, die in der Regel ein Vorschubglied, wie etwa eine Kolbenstange aufweist, welches gegenüber dem Gehäuse in Richtung der Aufnahmevorrichtung für das fluide Produkt vorgeschoben werden kann. Dort trifft es im Allgemeinen auf einen Stopfen der Aufnahmevorrichtung, so dass durch das Verschieben des Vorschubgliedes auch der Stopfen in der Aufnahmevorrichtung angetrieben und das fluide Produkt ausgeschüttet wird. Der Antrieb des Vorschubgliedes kann manuell erfolgen. Es ist jedoch auch möglich, einen Antrieb in Form einer vorgespannten Feder vorzusehen. Weiter sind zudem auch nicht mechanische Antriebe, wie z.B. Gasantriebe bekannt.

Das Betätigungselement zum Betätigen der Verabreichungseinrichtung ist gegenüber dem Gehäuse beweglich, um auf die Verabreichungseinrichtung wirken zu können. Das Betätigungselement kann in Form eines Knopfes aus dem Gehäuse herausragen. Es ist jedoch auch möglich, das Betätigungselement seitlich am Gehäuse anzubringen, z.B. in Form eines Schiebers. Mit dem Betätigungselement kann zum Betätigen der Verabreichungsmechanik, z.B. das Vorschubglied unmittelbar angetrieben werden, oder es kann ein vorgespanntes Federelement aus der Vorspannung gelöst werden, welches wiederum auf das Vorschubglied wirkt.

In der Aufnahmevorrichtung zur Aufnahme des fluiden Produkts ist das fluide Produkt untergebracht. Vorzugsweise befindet sich das fluide Produkt in einer Karpule, welche in die Aufnahmevorrichtung eingesetzt werden kann. Derartige Karpulen weisen in der Regel ein erstes Ende auf, welches durch einen Stopfen abgeschlossen ist und ein zweites Ende, das durch eine dünne Membran abgeschlossen ist, durch welche bei der Anwendung eine Kanüle einer Injektionsnadeleinheit durchgestochen werden kann. Die Aufnahmevorrichtung mit der Karpule kann an die Verabreichungsmechanik angeschlossen werden, in dem die Aufnahmevorrichtung in das Gehäuse eingesetzt oder an das dem Gehäuse angebracht wird.

Zum Blockieren des Betätigungselements weist die Vorrichtung zur Verabreichung eines fluiden Produkts eine Blockiereinrichtung auf. Die Blockiereinrichtung verhindert die Betätigung des Betätigungselements und somit die Betätigung der Verabreichungseinrichtung, wenn sich die Blockiereinrichtung in einer Blockierposition befindet. Die Blockiereinrichtung kann von der Blockierposition in eine Freigabeposition bewegt werden, in der das Betätigungselement zur Verabreichung des fluiden Produkts mittels der Verabreichungseinrichtung betätigt werden kann.

Gemäss der vorliegenden Erfindung ist die Aufnahmevorrichtung relativ zum Gehäuse drehbar gelagert, wobei durch Drehung der Aufnahmevorrichtung relativ zum Gehäuse die Blockiereinrichtung von der Blockierposition in die Freigabeposition bewegbar ist. Dabei wird die Aufnahmevorrichtung beispielsweise um eine Längsachse des Gehäuses, bzw. der Aufnahmevorrichtung, gedreht. Somit kann mittels der Drehung der Aufnahmevorrichtung der Pen entsichert und die Verabreichungseinrichtung mit dem Betätigungselement ausgelöst werden.

Eine derartige Entriegelung der Blockiereinrichtung ist insbesondere bei der Verwendung von Zweikammer-Karpulen vorteilhaft, welche für den Abmischvorgang in das Gehäuse der Verabreichungsvorrichtung eingedreht bzw. eingeschraubt werden. In diesem Fall kann mittels dem einen Drehvorgang z.B. zum einen das Abmischen in der Zweikammer-Karpule ausgelöst werden und zum anderen die Blockiereinrichtung der Verabreichungsvorrichtung in eine Freigabeposition bewegt werden. Möglich ist es auch, dass die Aufnahmevorrichtung mittels einem Bajonettverschluss an dem Gehäuse angeordnet wird, wobei auch der Bajonettverschluss durch eine Drehbewegung erzeugt wird. Auch bei dieser Drehung kann gemäss der vorliegenden Erfindung gleichzeitig die Blockiereinrichtung in eine Freigabeposition bewegt werden.

Um die Blockiereinrichtung von der Blockierposition in die Freigabeposition zu bewegen, weist vorzugsweise die Aufnahmevorrichtung einen Anschlag und die Blockiereinrichtung einen Gegenanschlag auf. Der Anschlag der Aufnahmevorrichtung und der Gegenanschlag der Blockiereinrichtung wirken derart zusammen, dass sie beim Drehen der Aufnahmevorrichtung gegenüber dem Gehäuse an einander anschlagen und bei einem weiteren Verdrehen der Aufnahmevorrichtung die Blockiereinrichtung mitnehmen und aus der Blockierposition in die Freigabeposition bewegen. Hierfür ist die Blockiereinrichtung z.B. relativ zum Gehäuse in Umfangsrichtung des Gehäuses verdrehbar.

Sobald sich die Blockiereinrichtung in der Freigabeposition befindet kann das Betätigungselement betätigt werden. Vorzugsweise wird das Betätigungselement in der Freigabeposition der Blockiereinrichtung entlang der Gehäuselängsachse relativ zum Gehäuse bewegt. Beispielsweise wird ein an einem Ende aus dem Gehäuse ragender Betätigungsknopf in das Gehäuse eingedrückt bzw. eingeschoben.

Gemäss einer ersten Ausführungsform der vorliegenden Erfindung ist die Blockiereinrichtung am Betätigungselement angeordnet. Beispielsweise können die Blockiereinrichtung und das Betätigungselement einstückig ausgebildet sein. In diesem Fall können die Blockiereinrichtung und das Betätigungselement aus einem Stück gefertigt werden. Es ist jedoch auch möglich, die Blockiereinrichtung nachträglich an dem Betätigungselement fest anzubringen. In einer Variante bewegt sich dann das Betätigungselement mit der Blockiereinrichtung mit, wenn die Blockiereinrichtung von der Blockierposition in die Freigabeposition bewegt wird. Beispielsweise wird das Betätigungselement beim Drehen der Aufnahmevorrichtung mitgedreht.

In einer anderen Variante ist die Blockiereinrichtung vorzugsweise als ein flexibler Arm ausgebildet. Der flexible Arm ist in Umfangsrichtung einer Achse des Betätigungselements auslenkbar, das heisst er kann sich in Drehrichtung um die Längsachse aus seiner Ruhelage heraus verbiegen. In dieser Ausführungsform bildet der flexible Arm den Gegenanschlag der Blockiereinrichtung, der mit dem Anschlag der Aufnahmeeinrichtung zusammenwirkt. Beim Verdrehen der Aufnahmevorrichtung schlägt deren Anschlag an dem flexiblen Arm an und lenkt den Arm aus der Ruhelage aus, wodurch die Blockiereinrichtung in eine Freigabeposition bewegt wird.

Das Blockieren des Betätigungselements wird vorzugsweise durch einen Blockieranschlag an der Blockiereinrichtung ausgeführt, der in der Blockierposition an einem Längsanschlag am Gehäuse, bzw. an einem in Längsrichtung gehäusefesten Teil, anschlägt. Als Längsanschlag soll ein Anschlag verstanden werden, der eine Bewegung entlang einer Längsachse der Verabreichungsvorrichtung blockiert. Liegt der Blockieranschlag an dem Längsanschlag an, kann das Betätigungselement nicht in dieser Längsrichtung betätigt werden. Ein Eindrücken oder Einschieben des Betätigungselements in das Gehäuse ist somit blockiert. Diese Blockierung wird durch das Auslenken des flexiblen Armes aufgehoben, indem der Blockieranschlag an der Blockiereinrichtung durch die Auslenkung des flexiblen Arms beim Drehen der Aufnahmeeinrichtung von dem Längsanschlag seitlich wegbewegt wird. In der ausgelegten Stellung des flexiblen Armes befindet sich die Blockiereinrichtung in einer Freigabeposition. Der Blockieranschlag kann dann seitlich in Längsrichtung an dem Längsanschlag vorbei geführt werden, wenn das Betätigungselement in das Gehäuse hineinbewegt wird.

In einer zweiten Ausführungsform der Erfindung ist die Blockiereinrichtung als Drehelement ausgebildet, welches relativ zum Betätigungselement gedreht werden kann. In diesem Fall weist die Blockiereinrichtung einen Blockieranschlag auf, der in der Blockierposition an einem Längsanschlag am Betätigungselement oder am Gehäuse, bzw. an einem gehäusefesten Teil, anschlägt. Das Betätigungselement ist auch in dieser Ausführung somit gegenüber der Blockiereinrichtung in der Blockierposition nicht in Längsrichtung des Gehäuses verschiebbar. Ist der Längsanschlag am Betätigungselement vorgesehen, wird das Drehelement derart drehbar im Gehäuse gelagert, dass es relativ zum Gehäuse nicht in Längsrichtung verschiebbar ist. Durch Verdrehen des Drehelements mittel der Drehung der Aufnahmevorrichtung wird der Blockieranschlag vom Längsanschlag am Betätigungselement entfernt und die Blockierung aufgehoben. Das Betätigungselement kann dann relativ zum Gehäuse und zum Drehelement in Längsrichtung verschoben werden.

Ist der Längsanschlag am Gehäuse oder einem gehäusefesten Teil vorgesehen, wird das Drehelement derart im Gehäuse gelagert, dass es in der Blockierposition relativ zum Gehäuse drehbar, aber nicht längsverschiebbar ist und in der Freigabeposition relativ zum Gehäuse in Längsrichtung verschoben werden kann. Die Blockierung wirkt demnach zwischen Blockiereinrichtung in Form des Drehelements und dem Gehäuse. Wird das Drehelement durch die Drehung der Aufnahmevorrichtung relativ zum Gehäuse verdreht, wird der Blockieranschlag vom Längsanschlag am Gehäuse oder dem gehäusefesten Teil wegbewegt. Die Blockiereinrichtung kann dann gemeinsam mit dem Betätigungselement relativ zum Gehäuse in Längsrichtung verschoben werden. Somit ist letztlich auch bei diesen Varianten das Betätigungselement an einer Betätigung gehindert. Die Blockiereinrichtung, bzw. das Drehelement, werden durch die Drehbewegung der Aufnahmevorrichtung relativ zum Gehäuse mittels dem Anschlag an der Aufnahmevorrichtung und dem Gegenanschlag an der Blockiereinrichtung verdreht. Durch diese Verdrehung wird der Anschlag zwischen dem Blockieranschlag der Blockiereinrichtung und dem Längsanschlag am Betätigungselement oder am Gehäuse, bzw. am gehäusefesten Teil, aufgehoben und diese Anschläge können in Längsrichtung an einander vorbei bewegt werden. Demnach befindet sich in dieser Position die Blockiereinrichtung in einer Freigabeposition in der das Betätigungselement betätigt werden kann.

Es ist vorteilhaft, wenn das Drehelement der Blockiereinrichtung in der Blockierposition gesichert ist. Eine derartige Sicherung kann z.B. durch einen Klemmsitz oder durch eine Vorspannung eines Federelements erfolgen. Beim Drehen der Aufnahmevorrichtung wird dann das Drehelement aus dem Klemmsitz geschoben, bzw. entgegen der Federspannung ausgelenkt.

Bei beiden Ausführungsformen der vorliegenden Erfindung ist eine Rasteinrichtung vorgesehen, welche die Aufnahmevorrichtung in der Freigabeposition der Blockiereinrichtung relativ zum Gehäuse verrastet. Durch die Verrastung wird sichergestellt, dass die Aufnahmevorrichtung nicht in der Gegenrichtung zurückdreht und somit die Blockiereinrichtung aus der Freigabeposition wieder zurück in eine Blockierposition bewegt wird. Eine solche Rasteinrichtung kann beispielsweise durch einen Schnapper an der Aufnahmevorrichtung realisiert werden, der in eine Vertiefung beispielsweise am Gehäuse einschnappt.

Besonders vorteilhaft ist die Anwendung der vorliegenden Erfindung beim Einsatz einer Zweikammer-Karpule in einer Vorrichtung zur Verabreichung eines fluiden Produktes. Die Aufnahmevorrichtung für die Zweikammer-Karpule weist ein Gewinde und das Gehäuse ein Gegengewinde auf Somit kann die Aufnahmevorrichtung in das Gehäuse eingedreht bzw. eingeschraubt werden. Beim Einsatz der Zweikammer-Karpule kann das Eindrehen der Aufnahmevorrichtung in das Gehäuse zum Abmischen der Komponenten in der Zweikammer-Karpule verwendet werden. Durch das Eindrehen der Aufnahmevorrichtung in das Gehäuse wird zunächst ein in der Zweikammer-Karpule z.B. mittels dem Vorschubglied der Verabreichungsmechanik vorgeschoben. Der Vorschub wird über das Lösungsmittel in der ersten Kammer auf den zweiten Stopfen übertragen der die Lösungsmittelkammer von der Wirkstoffkammer trennt. Die beiden Stopfen werden so lange gleichmässig verschoben, bis der zweite Stopfen an einem Bypass in der Karpulenwand angekommen ist, durch den das Lösungsmittel aus der Lösungsmittelkammer in die Wirkstoffkammer gelangen kann. Der erste Stopfen wird solange vorgeschoben bis das Lösungsmittel in der ersten Kammer ist und er am zweiten Stopfen zu liegen kommt. Vorzugsweise ist das Eindrehen der Zweikammer-Karpule derart vorgesehen, dass die Blockiereinrichtung von der Blockierposition in die Freigabeposition bewegt ist, sobald das Lösungsmittel vollständig aus der Lösungsmittelkammer in die Wirkstoffkammer gelangt ist. Mittels der Rasteinrichtung wird die Aufnahmevorrichtung in der Freigabeposition der Blockiereinrichtung relativ zum Gehäuse verrastet, so dass die Vorrichtung zur Verabreichung in dieser Stellung fertig für eine Injektion ist, das heisst, der Wirkstoff ist vollständig abgemischt und die Blockierung der Verabreichungsvorrichtung ist aufgehoben. Sobald eine Injektionsnadeleinheit auf die Aufnahmevorrichtung aufgesetzt ist, kann eine Injektion durchgeführt werden. Bei dieser Ausführungsform ist es vorteilhaft, dass kein gesonderter Handgriff zur Entriegelung der Blockiereinrichtung notwendig ist, sondern die Auflösung der Blockierung durch das ohnehin notwendige Abmischen der Zweikammer-Karpule durchgeführt wird.

### Kurze Beschreibung der Zeichnungen

Bevorzugte Ausführungsbeispiele der Erfindung werden nachfolgend Anhand der Figuren erläutert. An den Ausführungsbeispielen offenbar werdende Merkmale bilden je einzeln und in jeder Kombination die Gegenstände der Ansprüche und auch die vorstehend beschriebenen Ausgestaltungen in vorteilhafter Weise weiter. Es zeigen:
- Figur 1: eine Verabreichungsvorrichtung in einem Ausgangszustand,
- Figur 2: die Verabreichungsvorrichtung in einem abgemischten Zustand,
- Figur 3: die Verabreichungsvorrichtung in einem entlüfteten Zustand,
- Figur 4: die Verabreichungsvorrichtung in einem ausgelösten Zustand,
- Figur 5: die Verabreichungsvorrichtung in einem Zustand nach der Ausschüttung eines Produkts,
- Figur 6a: Innenansicht der Verabreichungsvorrichtung in einer Blockierposition,
- Figur 6b: Innenansicht der Verabreichungsvorrichtung in einer Freigabeposition,
- Figur 7a: Detailansicht einer zweiten Ausführungsform einer Verabreichungsvorrichtung in einer Blockierposition und
- Figur 7b: Detailansicht der zweiten Ausführungsform der Verabreichungsvorrichtung in einer Freigabeposition.

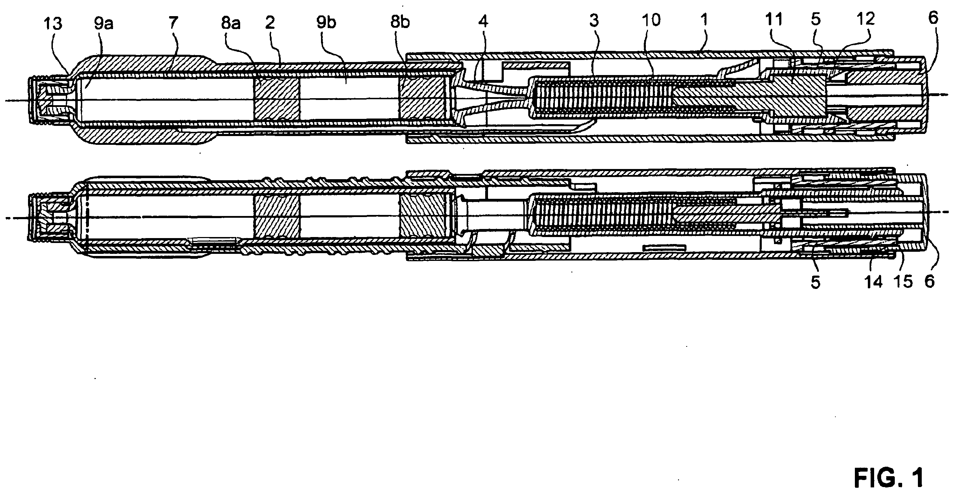

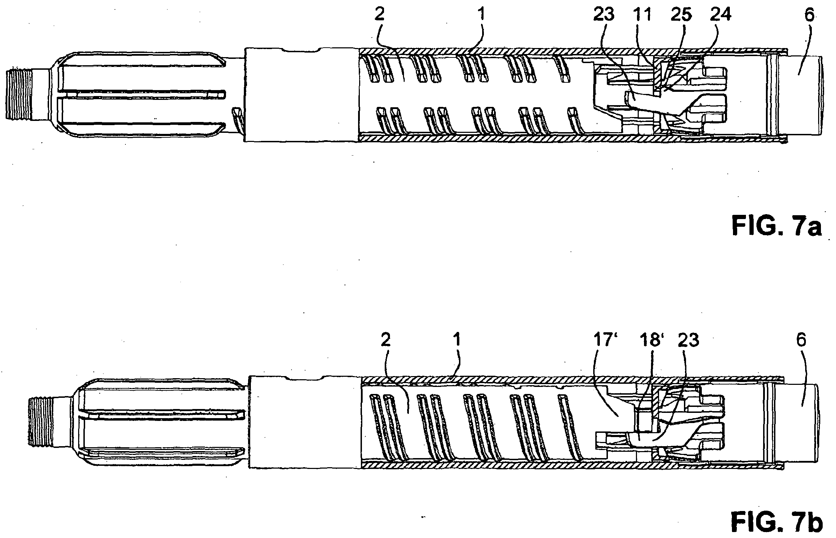

Die Figuren 1 bis 6 zeigen eine erste Ausführungsform einer Verabreichungsvorrichtung gemäss der vorliegenden Erfindung mit einer Blockiereinrichtung zum Blockieren eines Betätigungselements. In den Figuren 7a und 7b ist eine zweite Ausführungsform einer Blockiereinrichtung zum Blockieren eines Betätigungselements dargestellt. Die Figuren 1 bis 5 sind jeweils in zwei Ansichten gezeigt, wobei die zweite, untere Ansicht gegenüber der ersten, oberen Ansicht um 90° gedreht ist.

Das Verabreichungsgerät gemäss der ersten Ausführungsform verwendet eine Zweikammer-Karpule als Behälter für ein fluides Produkt. Die Ausschüttung des fluiden Produkts aus der Karpule erfolgt durch den Vorschub eines Antriebsglieds mittels einer Ausschüttfeder. Es erfolgt daher eine automatische Ausschüttung des fluiden Produkts, sobald die Ausschüttfeder aktiviert wird. Die Verabreichungsvorrichtung weist eine fixe Dosierung auf, das heisst, das Ausschüttvolumen ist fest vorgegeben. Der Vorschub des Antriebsglieds ist daher ebenfalls fest vorgegeben und kann nicht individuell eingestellt werden. Das Verabreichungsgerät ist nach einer einzigen Ausschüttung blockiert und wird nach der Ausschüttung entsorgt.

Für einen Fachmann ist es klar, dass eine Blockiereinrichtung gemäss der vorliegenden Erfindung gleichwohl bei wieder verwendbaren Verabreichungsgeräten, bei Geräten mit individueller Dosierung oder manueller Ausschüttung und auch bei Geräten mit Einkammer-Karpulen vorteilhaft eingesetzt werden kann.

Im Folgenden wird mit den Begriffen distal, bzw. proximal, das Ende des Verabreichungsgeräts, an welchem das fluide Produkt ausgeschüttet wird, bzw. das gegenüberliegende Ende, bezeichnet.

Das Verabreichungsgerät gemäss der ersten Ausführungsform der vorliegenden Erfindung weist ein Gehäuse 1, eine Aufnahmevorrichtung zur Aufnahme des fluiden Produkts in Form eines Karpulenhalters 2, ein Antriebsglied 3 mit einer Halteeinrichtung in Form von Haltearmen 4, eine Blockiereinrichtung in Form eines Blockierrings 5 und ein Betätigungselements in Form eines Auslöseknopfes 6 auf.

In dem Karpulenhalter 2 ist eine Zweikammer-Karpule 7 untergebracht. Die Zweikammer-Karpule weist einen ersten Stopfen 8a und einen zweiten Stopfen 8b auf. Der zweite Stopfen 8b schliesst die Zweikammer-Karpule am proximalen Ende ab. Am distalen Ende weist die Zweikammer-Karpule eine Verjüngung auf, deren Öffnung durch eine Membran abgeschlossen ist. Die Membran kann durch eine Kanüle einer Injektionsnadeleinheit durchstochen werden. Die Injektionsnadeleinheit wird in den Figuren nicht dargestellt. Zwischen der Membran und dem ersten Stopfen 8a ist eine erste Kammer 9a ausgebildet, in welcher ein trockener bzw. lyophilisierter Wirkstoff untergebracht wird (nicht dargestellt). Zwischen dem ersten Stopfen 8a und dem zweiten Stopfen 8b wird eine zweite Kammer gebildet, in welcher das Lösungsmittel für den Wirkstoff gelagert wird.

Das Antriebsglied 3 ist hülsenartig ausgebildet. Im inneren des Antriebsglieds 3 ist eine Antriebsfeder 10 angeordnet, die zwischen einem distalen Anschlag am Hülsenboden des Antriebsglieds und einem proximalen Anschlag an einem gehäusefesten Element 11 eingespannt ist. Im Ausgangszustand der Figur 1 wird das Antriebsglied 3 relativ zum Gehäuseelement 11 durch Schnapparme 12 gehalten, welche hinter einem Anschlag des Gehäuseelements 11 lösbar einschnappen. Am distalen Ende des Antriebsglieds 3 sind die Haltearme 4 derart angebracht, dass sie in dieser Ausgangsposition von einer Längsachse des Abtriebsglieds seitlich abragen. In der gezeigten Ausführungsform sind zwei Haltearme dargestellt, die zueinander aufgespreizt sind. Es können natürlich auch drei oder mehr derartige Haltearme 4 vorgesehen werden. In dem Ausgangszustand in Figur 1 stossen die Haltearme 4 mit ihrem distalen Ende an eine proximale Kante der Karpule 7. Die Haltearme 4 drücken die Karpule 7 gegen eine Schulter 13 des Karpulenhalters 2. Die Karpule 7 wird daher von der Halteeinrichtung in Form der Haltearme 4 in einer definierten Position relativ zum Karpulenhalter 2 gehalten. Eine hin und her Bewegung in proximaler oder distaler Richtung der Karpule im Halter wird dadurch unterbunden.

Der Blockierring 5 befindet sich im Ausgangszustand der Figur 1 in einer Blockierposition, in der er eine Betätigung des Auslöseknopfes 6 blockiert, d.h. der Auslöseknopf 6 kann nicht in Längsrichtung in das Gehäuse 1 eingedrückt werden. Hierfür weist der Blockierring 5 einen Blockieranschlag 14 auf, der an einem Gegenanschlag 15 am Auslöseknopf 6 ansteht. Aufgrund des Aneinanderstossens des Blockieranschlags 14 des Blockierrings 5 und des Gegenanschlags 15 des Auslöseknopfes 6 kann der Auslöseknopf 6 nicht betätigt werden, das heisst er kann nicht relativ zum Gehäuse entlang der Längsachse in das Gehäuse eingedrückt werden. Hierfür ist der Blockierring 5 relativ zum Gehäuse in Längsrichtung festgelagert, kann jedoch relativ zum Gehäuse verdreht werden. Der Blockieranschlag 14 kann z.B. durch Rippen oder Nocken am Blockierring oder durch die proximale Kante des Blockierrings 5 realisiert werden.

Der Blockierring 15 ist hülsenartig ausgebildet und umgibt die Schnapparme 12 des Antriebsglieds 3. Im Ausgangszustand der Figur 1 liegt die Innenumfangsfläche des Blockierrings 5 derart aussen an den Schnapparmen 12 an, dass diese nicht aus ihrer Verschnappung hinter dem Gehäuseelement 11 freigegeben werden können. Der Blockierring 5 blockiert somit zum einen eine Betätigung des Auslöseknopfes 6 und zum anderen eine Freigabe der Schnapparme 12. Der Ausgangszustand entspricht einem Auslieferungszustand, wie das Verabreichungsgerät an einen Anwender abgegeben wird. Eine Betätigung des Verabreichungsgeräts ist in diesem Zustand nicht möglich.

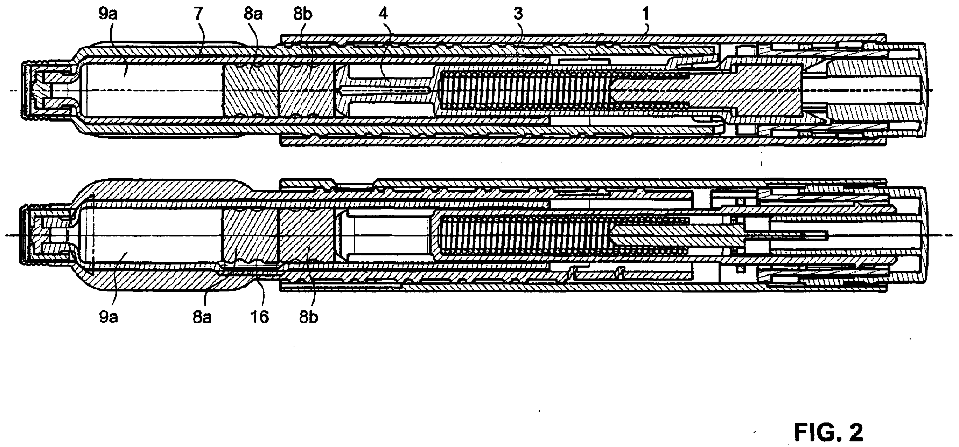

In Figur 2 ist das Verabreichungsgerät in einem abgemischten Zustand gezeigt, in dem der Wirkstoff der Kammer 9a der Zweikammer-Karpule 7 mit dem Lösungsmittel der Kammer 9b abgemischt ist. Die Beendigung des Abmischens kann durch ein taktiles, akustisches oder visuelles Signal angezeigt werden. Wie in Figur 2 gezeigt ist wurden zum Abmischen die Stopfen 8a und 8b innerhalb der Karpule 7 verschoben, bis der Stopfen 8a an einem Bypass 16 zu liegen kommt, durch den das Lösungsmittel in die Kammer 9a fliessen kann und der Stopfen 8b an dem Stopfen 8a zu liegen kommt. Zum Vorschub der Stopfen wird der Karpulenhalter 2 in das Gehäuse 1 eingeschraubt, so dass das Antriebsglied, dass in diesem Zustand relativ zum Gehäuse in Ruhe ist, die Stopfen 8a und 8b relativ zur Karpule 7 verschiebt. Zum Einschrauben des Karpulenhalters ist an der Innenseite des Gehäuses ein Innengewinde und an der Aussenseite des Karpulenhalters ein Aussengewinde vorgesehen.

Wie in Figur 2 zu sehen ist, sind die Haltearme 4 an der proximalen Kante der Karpule 7 abgerutscht und wurden radial nach innen in Richtung der Längsachse des Antriebsglieds bewegt. Hierfür weisen die Enden der Haltearme 4 schräge Flächen auf, entlang welcher die Haltearme 4 nach innen abgelenkt werden, sobald die proximale Kante der Karpule 7 mit ausreichender Kraft gegen die schrägen Flächen gedrückt wird, wie es beim Einschrauben des Karpulenhalters 2 in das Gehäuse 1 der Fall ist. Die Haltearme 4 bewegen sich dabei nach innen aufeinander zu und bilden einen Stössel für den Stopfen 8b der Karpule 7. Durch den Anschlag der Haltearme 4 an dem Stopfen 8b wird die Karpule 7 weiterhin in ihrer definierten Position in dem Karpulenhalter 2 gehalten, während die Stopfen 8a und 8b innerhalb der Karpule 7 verschoben werden. Unabhängig davon bilden die Haltearme 4 mit der Innenwand der Karpule 7 eine Klemmpassung, bzw. einen Klemmsitz, da sie eine Vorspannung in radialer Richtung nach aussen aufweisen, seitdem sie radial nach innen verbogen wurden. Diese Klemmpassung dient dem Halten der Karpule in einer definierten Position in dem Karpulenhalter.

Nach dem Abmischen der Zweikammer-Karpule kann es notwendig sein, die Kammer 9a mit dem gelösten Wirkstoff zu entlüften, bevor der Wirkstoff injiziert werden kann. Hierfür wird eine Injektionsnadeleinheit auf den Karpulenhalter 2 am distalen Ende derart aufgebracht, dass eine Kanüle die Membran der Karpule 2 durchsticht und somit eine Flüssigkeitsverbindung zur Kammer 9a herstellt. Geringfügiges weiteres Eindrehen des Karpulenhalters 7 führt zu einem weitem Vorschub der Stopfen 8a und 8b, so dass in der Kammer 9a befindliche Luft entweichen kann. Ein Vorschub wird normalerweise so lange durchgeführt, bis eine geringe Menge des Wirkstoffes 9a aus der Kanüle der Injektionsnadeleinheit austritt. Der Abschluss der Entlüftung kann durch ein taktiles, akustisches oder visuelles Signal angezeigt werden.

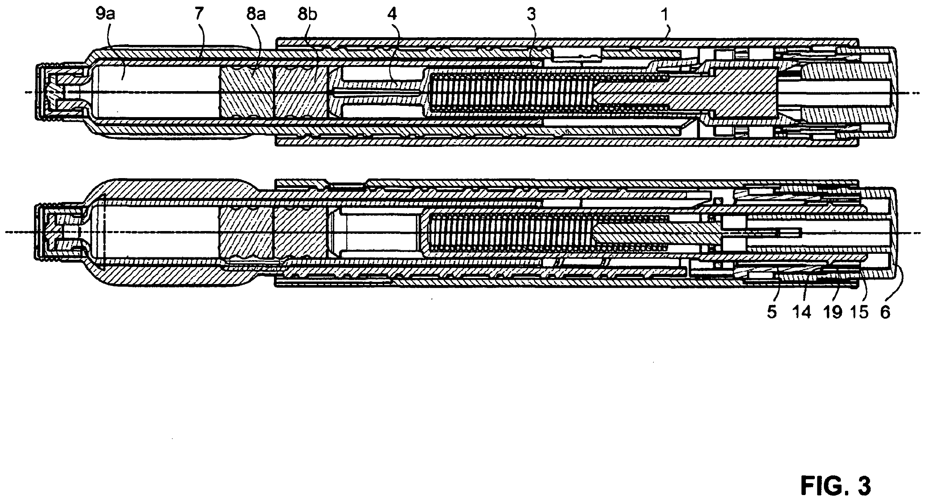

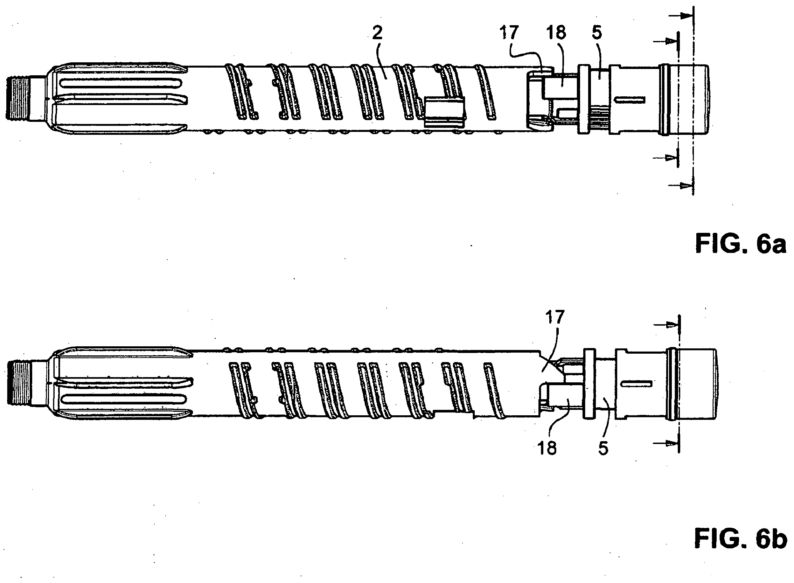

Dieser entlüftete Zustand ist in Figur 3 dargestellt. Auf die Darstellung der Injektionsnadeleinheit wurde aus Gründen der Einfachheit verzichtet. Bei der letzten Einschraubbewegung des Karpulenhalters 2 in das Gehäuse 1, bei dem auch die Karpule entlüftet werden kann, wird der Blockierring 5 von der Blockierposition in eine Freigabeposition bewegt. Wie in den Figuren 6a bis 6d gezeigt ist, weist hierfür der Karpulenhalter einen Anschlag 17 und der Blockierring einen Gegenanschlag 18 auf. Der Anschlag 17 des Karpulenhalters 2 ist derart ausgebildet, dass er bei der Drehbewegung des Karpulenhalters in Umfangsrichtung gegen den Gegenanschlag 18 des Blockierrings 5 anstösst. Bei einem weiteren Verdrehen des Karpulenhalters 2 nimmt der Karpulenhalter den Blockierrings 5 mit, so dass dieser relativ zum Gehäuse 1 und zum Auslöseknopf 6 gedreht wird. Durch diese Drehbewegung wird der Blockierring von der Blockierposition in die Freigabeposition bewegt. Wie in Figur 3 dargestellt ist, wird bei der Drehung der Blockieranschlag 14 des Blockierrings 5 von dem Gegenanschlag 15 des Auslöseknopfes 6 weggedreht, bis dem Gegenanschlag 15 eine Nut oder Rille 19 des Blockierrings gegenüberliegt, innerhalb der der Gegenanschlag 15 des Auslöseknopfes 6 in Längsrichtung verschoben werden kann.

Beim Drehen des Blockierrings 5 mittels dem Karpulenhalter 2 werden zudem die Innenflächen des Blockierrings 5, welche die Schnapparme 12 an einem Ausschnappen aus ihrer Schnappposition hindern, aus dieser Position weggedreht. In der Freigabeposition des Blockierrings 5 liegen den Schnapparmen 12 Aussparungen in der Hülsenfläche des Blockierrings 5 gegenüber. Auch gegenüber den Schnapparmen 12 befindet sich der Blockierring 5 demnach in einer Freigabeposition.

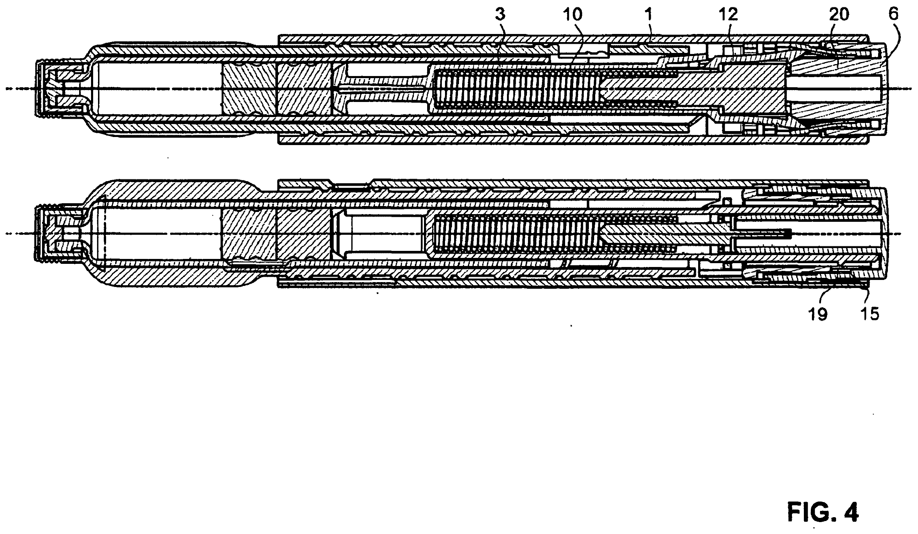

In Figur 4 ist die Verabreichungsvorrichtung in einem ausgelösten Zustand dargestellt, in dem der Auslöseknopf 6 relativ zum Gehäuse 1 entlang der Längsachse in das Gehäuse 1 eingedrückt wurde. Dabei wurden die Gegenanschläge 15 des Auslöseknopfes 6 innerhalb der Rillen 19 des Blockierrings 5 verschoben. Der Auslöseknopf 6 weist nach innen ragende Stege 20 auf, die in dem ausgelösten, bzw. eingeschobenen Zustand des Auslöseknopfes gegen schräge Flächen am proximalen Ende der Schnapparme 12 anliegen und diese beim Vorschub des Auslöseknopfes 6 radial nach aussen aufspreizen, so dass die Enden der Schnapparme innerhalb der Aussparungen im Blockierring 5 zu liegen kommen. Durch das Aufspreizen der Schnapparme 12 wird die Sicherung des Antriebsglieds 3 am Gehäuseelement 11 gelöst. In dem ausgelösten Zustand beginnt die Federkraft der Antriebsfeder 10 zu wirken und drückt gegen das Antriebsglied 3.

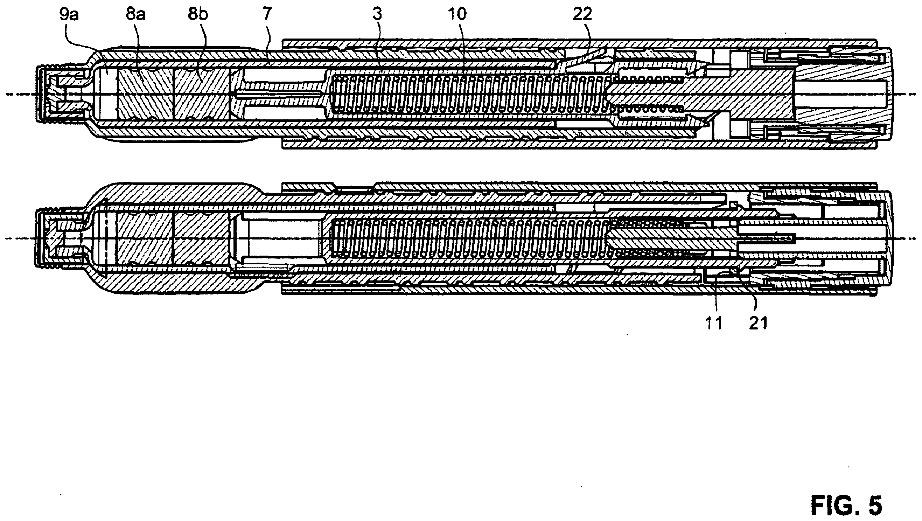

Wie in Figur 5 gezeigt ist, wird das Antriebsglied 3 durch die Kraft der Feder 10 relativ zur Karpule 7 vorgeschoben und treibt die Stopfen 8a und 8b innerhalb der Karpule 7 an, so dass der Wirkstoff aus der Kammer 9a ausgeschüttet wird. Die Antriebsfeder 10 schiebt das Antriebsglied 3 so lange in die Karpule vor, bis ein Vorsprung 21, der am Antriebsglied 3 vorgesehen ist, an einer Kante des Gehäuseelements 11 anschlägt. Sobald der Vorsprung 21 am Gehäuseelement 11 anschlägt, ist die Ausschüttung des Wirkstoffes beendet.

In dem dargestellten Ausführungsbeispiel ist an dem Antriebsglied 3 ein flexibler Arm 22 vorgesehen, der bei Beendigung der Ausschüttung in eine Aussparung im Karpulenhalter hineinragt und als Blockierung für eine Bewegung des Antriebsglieds 3 in proximaler Richtung dient. Ferner erzeugt der Arm 22 beim Einrasten in die Aussparung des Karpulenhalters ein akustisches Geräusch, durch welches angezeigt wird, dass die Ausschüttung beendet ist.

In den Figuren 7a und 7b ist eine zweite Ausführungsform einer Verabreichungsvorrichtung mit einer erfindungsgemässen Blockiereinrichtung gezeigt. In dieser Variante ist die Blockiereinrichtung am Betätigungselement in Form des Auslöseknopfes angeordnet. Wie in Figur 7a gezeigt ist, ragt von dem Auslöseknopf 6 in Längsrichtung der Verabreichungsvorrichtung ein Verriegelungsarm 23 ab. Der Verriegelungsarm 23 und der Auslöseknopf 6 sind einstückig ausgebildet. An dem Verriegelungsarm 23 ist ein Blockieranschlag 24 vorgesehen, der an einem Längsanschlag des Gehäuseelements 11 in Längsrichtung anschlägt. Durch die Blockierung des Blockieranschlags 24 am Längsanschlag 25 kann der Auslöseknopf 6 nicht in Längsrichtung in das Gehäuse 1 eingedrückt werden.

In Figur 7a ist der Karpulenhalter 2 bereits in das Gehäuse 1 eingeschraubt. Der Karpulenhalter 2 wird so lange in das Gehäuse eingeschraubt, bis ein Anschlag 17' des Karpulenhalters in Umfangsrichtung gegen einen Gegenanschlag 18' des Verriegelungsarms 23 des Auslöseknopfes 6 stösst. Beim Weiterdrehen des Karpulenhalters 2 wird der Verriegelungsarm 23 in Umfangsrichtung gegenüber seiner Ruhelage ausgelenkt, da der Anschlag 17' gegen den Gegenanschlag 18' wirkt. Dadurch wird der Blockieranschlag des Auslöseknopfes 6 aus seiner Blockierposition gegenüber dem Längsanschlag 25 des Gehäuseelements 11 ausgelenkt und kommt gegenüber einer Aussparung im Gehäuseelement 11 zu liegen. Die Blockiereinrichtung zum Blockieren des Auslöseknopfes 6 befindet sich nun in einer Freigabeposition, in der der Auslöseknopf 6 entlang der Längsachse in das Gehäuse 1 eingedrückt werden kann. Dabei wird der Blockieranschlag 24 durch die Aussparung im Gehäuseelement 11 geführt.

Bei dieser Ausführungsform weist der Karpulenhalter 2 eine Rasteinrichtung auf, mit der er gegenüber dem Gehäuse verrastet, sobald die Blockiereinrichtung in einer Freigabeposition ist, damit ein Zurückdrehen des Karpulenhalters und somit eine erneute Blockierung des Auslöseknopfes verhindert wird.

### Bezugszeichenliste

- 1: Gehäuse
- 2: Karpulenhalter
- 3, 3': Antriebsglied
- 4, 4': Haltearm
- 5: Blockierring
- 6: Auslöseknopf
- 7: Zweikammer-Karpule
- 8a, b: Stopfen
- 9a, b: Kammer
- 10: Antriebsfeder
- 11: gehäusefestes Element
- 12: Schnapparme
- 13: Schulter
- 14: Blockieranschlag
- 15: Gegenanschlag
- 16: Bypass
- 17, 17': Anschlag
- 18, 18': Gegenanschlag
- 19: Rille
- 20: Steg
- 21: Vorsprung
- 22: Arm
- 23: Verriegelungsarm
- 24: Blockieranschlag
- 25: Längsanschlag
- 26: Stössel

## Patentansprüche

1. Vorrichtung zur Verabreichung eines fluiden Produkts umfassend:
- ein Gehäuse (1) zur Aufnahme einer Verabreichungseinrichtung,
- ein Betätigungselement (6) zum Betätigen der Verabreichungseinrichtung,
- eine Aufnahmevorrichtung (2) zur Aufnahme des fluiden Produkts,
- eine Blockiereinrichtung (5, 23) zum Blockieren des Betätigungselements (6), wobei
- die Aufnahmevorrichtung (2) relativ zum Gehäuse (1) drehbar gelagert ist,
- wobei durch Drehung der Aufnahmevorrichtung (2) relativ zum Gehäuse (1) die Blockiereinrichtung (5, 23) von einer Blockierposition in eine Freigabeposition bewegbar ist,
wobei eine Rasteinrichtung vorgesehen ist, welche die Aufnahmevorrichtung in der Freigabeposition der Blockiereinrichtung relativ zum Gehäuse verrastet, sodass die Aufnahmevorrichtung nur in eine Drehrichtung drehbar ist, um die Blockiereinrichtung (5, 23) von der Blockierposition in die Freigabeposition zu bewegen, und in der Freigabeposition nicht in die Gegenrichtung zurückgedreht werden kann,
**dadurch gekennzeichnet, dass**
die Aufnahmevorrichtung (7) einen Anschlag (17, 17') und die Blockiereinrichtung (5, 23) einen Gegenanschlag (18, 18') aufweist, die zur Bewegung der Blockiereinrichtung (5, 23) aus der Blockierposition in die Freigabeposition zusammenwirken.

2. Vorrichtung zur Verabreichung eines fluiden Produkts nach Anspruch 1, **dadurch gekennzeichnet, dass** das Betätigungselement (6) in der Freigabeposition der Blockiereinrichtung (5, 23) entlang einer Gehäuselängsachse relativ zum Gehäuse (1) beweglich ist.

3. Vorrichtung zur Verabreichung eines fluiden Produkts nach einem der vorhergehenden Ansprüche, **dadurch gekennzeichnet, dass** die Blockiereinrichtung (23) am Betätigungselement (6) angeordnet ist.

4. Vorrichtung zur Verabreichung eines fluiden Produkts nach Anspruch 3, **dadurch gekennzeichnet, dass** die Blockiereinrichtung (23) und das Betätigungselement (6) einstückig ausgebildet sind.

5. Vorrichtung zur Verabreichung eines fluiden Produkts nach einem der Ansprüche 3 oder 4, **dadurch gekennzeichnet, dass** die Blockiereinrichtung (23) einen Blockieranschlag (24) aufweist, der in der Blockierposition an einem Längsanschlag (25) am Gehäuse (1), bzw. einem in Längsrichtung gehäusefestem Teil (11), anschlägt.

6. Vorrichtung zur Verabreichung eines fluiden Produkts umfassend:
- ein Gehäuse (1) zur Aufnahme einer Verabreichungseinrichtung,
- ein Betätigungselement (6) zum Betätigen der Verabreichungseinrichtung,
- eine Aufnahmevorrichtung (2) zur Aufnahme des fluiden Produkts,
- eine Blockiereinrichtung (5, 23) zum Blockieren des Betätigungselements (6), wobei die Blockiereinrichtung (23) am Betätigungselement (6) angeordnet ist, wobei
- die Aufnahmevorrichtung (2) relativ zum Gehäuse (1) drehbar gelagert ist,
- wobei durch Drehung der Aufnahmevorrichtung (2) relativ zum Gehäuse (1) die Blockiereinrichtung (5, 23) von einer Blockierposition in eine Freigabeposition bewegbar ist,
wobei eine Rasteinrichtung vorgesehen ist, welche die Aufnahmevorrichtung in der Freigabeposition der Blockiereinrichtung relativ zum Gehäuse verrastet, sodass die Aufnahmevorrichtung nur in eine Drehrichtung drehbar ist, um die Blockiereinrichtung (5, 23) von der Blockierposition in die Freigabeposition zu bewegen, und in der Freigabeposition nicht in die Gegenrichtung zurückgedreht werden kann,
**dadurch gekennzeichnet, dass**
die Blockiereinrichtung einen flexiblen Arm (23) aufweist, der in Umfangsrichtung der Längsachse relativ zum Betätigungselement (6) auslenkbar ist.

7. Vorrichtung zur Verabreichung eines fluiden Produkts nach Anspruch 1 oder 2, **dadurch gekennzeichnet, dass** die Blockiereinrichtung (5) relativ zum Betätigungselement (6) drehbar angeordnet ist.

8. Vorrichtung zur Verabreichung eines fluiden Produkts nach dem vorhergehenden Anspruch, **dadurch gekennzeichnet, dass** die Blockiereinrichtung (5) einen Blockieranschlag (14) aufweist, der in der Blockierposition an einem Längsanschlag (15) am Betätigungselement (6) oder am Gehäuse (1) bzw. einem gehäusefesten Teil (11), anschlägt.

9. Vorrichtung zur Verabreichung eines fluiden Produkts nach einem der Ansprüche 7 oder 8, **dadurch gekennzeichnet, dass** die Blockiereinrichtung (5) ein Verriegelungsmittel umfasst, das in der Blockierposition ein Antriebsglied (3) gegen Vorschub verriegelt.

10. Vorrichtung zur Verabreichung eines fluiden Produkts umfassend:
- ein Gehäuse (1) zur Aufnahme einer Verabreichungseinrichtung,
- ein Betätigungselement (6) zum Betätigen der Verabreichungseinrichtung,
- eine Aufnahmevorrichtung (2) zur Aufnahme des fluiden Produkts,
- eine Blockiereinrichtung (5, 23) zum Blockieren des Betätigungselements (6), wobei
- die Aufnahmevorrichtung (2) relativ zum Gehäuse (1) drehbar gelagert ist,
- wobei durch Drehung der Aufnahmevorrichtung (2) relativ zum Gehäuse (1) die Blockiereinrichtung (5, 23) von einer Blockierposition in eine Freigabeposition bewegbar ist,
wobei eine Rasteinrichtung vorgesehen ist, welche die Aufnahmevorrichtung in der Freigabeposition der Blockiereinrichtung relativ zum Gehäuse verrastet, sodass die Aufnahmevorrichtung nur in eine Drehrichtung drehbar ist, um die Blockiereinrichtung (5, 23) von der Blockierposition in die Freigabeposition zu bewegen, und in der Freigabeposition nicht in die Gegenrichtung zurückgedreht werden kann,
**dadurch gekennzeichnet, dass**
die Aufnahmevorrichtung (2) eine Zweikammer-Karpule (7) aufnimmt, wobei die Zweikammer-Karpule (7) durch Drehung der Aufnahmevorrichtung (2) abmischbar ist.

11. Vorrichtung zur Verabreichung eines fluiden Produkts nach Anspruch 10, **dadurch gekennzeichnet, dass** die Freigabestellung der Blockiereinrichtung (5, 23) einer Stellung der Zweikammer-Karpule (7) entspricht, in der die Zweikammer-Karpule (7) abgemischt ist.

## Claims

1. A device for administering a fluid product, comprising:
- a housing (1) for accommodating an administering device;
- an actuating element (6) for actuating the administering device;
- an accommodating device (2) for accommodating the fluid product;
- a blocking means (5, 23) for blocking the actuating element (6), wherein
- the accommodating device (2) is mounted such that it can be rotated relative to the housing (1),
- wherein the blocking means (5, 23) can be moved from a blocking position to a release position by rotating the accommodating device (2) relative to the housing (1),
wherein a locking means is provided which locks the accommodating device relative to the housing when the blocking means is in its release position, such that the accommodating device can only be rotated in one rotational direction in order to move the blocking means (5, 23) from the blocking position to the release position, and cannot be rotated back in the opposite direction in the release position,
**characterised in that**
the accommodating device (7) comprises an abutment (17, 17'), and the blocking means (5, 23) comprises a complementary abutment (18, 18'), which co-operate to move the blocking means (5, 23) from the blocking position to the release position.

2. The device for administering a fluid product according to claim 1, **characterised in that** when the blocking means (5, 23) is in its release position, the actuating element (6) can be moved relative to the housing (1) along a longitudinal axis of the housing.

3. The device for administering a fluid product according to any one of the preceding claims, **characterised in that** the blocking means (23) is arranged on the actuating element (6).

4. The device for administering a fluid product according to claim 3, **characterised in that** the blocking means (23) and the actuating element (6) are formed in one piece.

5. The device for administering a fluid product according to any one of claims 3 or 4, **characterised in that** the blocking means (23) comprises a blocking abutment (24) which, in the blocking position, abuts against a longitudinal abutment (25) on the housing (1) or a part (11) which is fixed relative to the housing in the longitudinal direction.

6. A device for administering a fluid product, comprising:
- a housing (1) for accommodating an administering device;
- an actuating element (6) for actuating the administering device;
- an accommodating device (2) for accommodating the fluid product;
- a blocking means (5, 23) for blocking the actuating element (6), wherein the blocking means (23) is arranged on the actuating element (6), wherein
- the accommodating device (2) is mounted such that it can be rotated relative to the housing (1),
- wherein the blocking means (5, 23) can be moved from a blocking position to a release position by rotating the accommodating device (2) relative to the housing (1),
wherein a locking means is provided which locks the accommodating device relative to the housing when the blocking means is in its release position, such that the accommodating device can only be rotated in one rotational direction in order to move the blocking means (5, 23) from the blocking position to the release position, and cannot be rotated back in the opposite direction in the release position,
**characterised in that**
the blocking means comprises a flexible arm (23) which can be deflected relative to the actuating element (6) in the circumferential direction of the longitudinal axis.

7. The device for administering a fluid product according to claim 1 or 2, **characterised in that** the blocking means (5) is arranged such that it can be rotated relative to the actuating element (6).

8. The device for administering a fluid product according to the preceding claim, **characterised in that** the blocking means (5) comprises a blocking abutment (14) which, in the blocking position, abuts against a longitudinal abutment (15) on the actuating element (6) or on the housing (1) or a part (11) which is fixed relative to the housing.

9. The device for administering a fluid product according to any one of claims 7 or 8, **characterised in that** the blocking means (5) comprises a latching means which, in the blocking position, latches a drive member (3) against advancing.

10. A device for administering a fluid product, comprising:
- a housing (1) for accommodating an administering device;
- an actuating element (6) for actuating the administering device;
- an accommodating device (2) for accommodating the fluid product;
- a blocking means (5, 23) for blocking the actuating element (6), wherein
- the accommodating device (2) is mounted such that it can be rotated relative to the housing (1),
- wherein the blocking means (5, 23) can be moved from a blocking position to a release position by rotating the accommodating device (2) relative to the housing (1),
wherein a locking means is provided which locks the accommodating device relative to the housing when the blocking means is in its release position, such that the accommodating device can only be rotated in one rotational direction in order to move the blocking means (5, 23) from the blocking position to the release position, and cannot be rotated back in the opposite direction in the release position,
**characterised in that**
the accommodating device (2) accommodates a two-chamber carpule (7), wherein the two-chamber carpule (7) can be mixed by rotating the accommodating device (2).

11. The device for administering a fluid product according to claim 10, **characterised in that** the release position of the blocking means (5, 23) corresponds to a position of the two-chamber carpule (7) in which the two-chamber carpule (7) is mixed.

## Revendications

1. Dispositif pour administrer un produit fluide, comprenant :
- un boîtier (1) pour recevoir un dispositif d'administration ;
- un élément d'actionnement (6) pour actionner le dispositif d'administration ;
- un dispositif de réception (2) pour recevoir le produit fluide ;
- un dispositif de blocage (5, 23) pour bloquer l'élément d'actionnement (6),
- le dispositif de réception (2) étant monté de manière rotative par rapport au boîtier (1),
- le dispositif de blocage (5, 23) étant déplaçable depuis une position de blocage jusqu'à une position de déblocage en faisant tourner le dispositif de réception (2) par rapport au boîtier (1),
un dispositif d'arrêt étant prévu qui arrête le dispositif de réception par rapport au boîtier lorsque le dispositif de blocage est dans sa position de déblocage, de sorte que le dispositif de réception ne peut être tourné que dans une seule direction de rotation, afin de déplacer le dispositif de blocage (5, 23) depuis la position de blocage jusqu'à la position de déblocage, et ne peut pas être tourné encore en direction opposée dans la position de déblocage,
**caractérisé en ce que**
le dispositif de réception (7) comporte une butée (17, 17'), et le dispositif de blocage (5, 23) comporte une butée complémentaire (18, 18'), qui coopèrent pour déplacer le dispositif de blocage (5, 23) depuis la position de blocage jusqu'à la position de déblocage.

2. Dispositif pour administrer un produit fluide selon la revendication 1, **caractérisé en ce que** l'élément d'actionnement (6) est mobile par rapport au boîtier (1) le long d'un axe longitudinal du boîtier lorsque le dispositif de blocage (5, 23) est dans sa position de déblocage.

3. Dispositif pour administrer un produit fluide selon l'une quelconque des revendications précédentes, **caractérisé en ce que** le dispositif de blocage (23) est disposé sur l'élément d'actionnement (6).

4. Dispositif pour administrer un produit fluide selon la revendication 3, **caractérisé en ce que** le dispositif de blocage (23) et l'élément d'actionnement (6) sont formés d'une seule pièce.

5. Dispositif pour administrer un produit fluide selon l'une quelconque des revendications 3 ou 4, **caractérisé en ce que** le dispositif de blocage (23) comporte une butée de blocage (24) qui, en position de blocage, s'applique contre une butée longitudinale (25) du boîtier (1) ou d'une pièce (11) fixée par rapport au boîtier en direction longitudinale.

6. Dispositif pour administrer un produit fluide, comprenant :
- un boîtier (1) pour recevoir un dispositif d'administration ;
- un élément d'actionnement (6) pour actionner le dispositif d'administration ;
- un dispositif de réception (2) pour recevoir le produit fluide ;
- un dispositif de blocage (5, 23) pour bloquer l'élément d'actionnement (6), le dispositif de blocage (23) étant disposé sur l'élément d'actionnement (6),
- le dispositif de réception (2) étant monté de manière rotative par rapport au boîtier (1),
- le dispositif de blocage (5, 23) étant déplaçable depuis une position de blocage jusqu'à une position de déblocage en faisant tourner le dispositif de réception (2) par rapport au boîtier (1),
un dispositif d'arrêt étant prévu qui arrête le dispositif de réception par rapport au boîtier lorsque le dispositif de blocage est dans sa position de déblocage, de sorte que le dispositif de réception ne peut être tourné que dans une seule direction de rotation, afin de déplacer le dispositif de blocage (5, 23) depuis la position de blocage jusqu'à la position de déblocage, et ne peut pas être tourné encore en direction opposée dans la position de déblocage,
**caractérisé en ce que**
le dispositif de blocage comporte un bras flexible (23) qui peut être dévié par rapport à l'élément d'actionnement (6) en direction circonférentielle de l'axe longitudinal.

7. Dispositif pour administrer un produit fluide selon la revendication 1 ou 2, **caractérisé en ce que** le dispositif de blocage (5) est disposé de manière rotative par rapport à l'élément d'actionnement (6).

8. Dispositif pour administrer un produit fluide selon la revendication précédente, **caractérisé en ce que** le dispositif de blocage (5) comporte une butée de blocage (14) qui, en position de blocage, s'applique contre une butée longitudinale (15) de l'élément d'actionnement (6) ou du boîtier (1) ou d'une pièce (11) fixée par rapport au boîtier.

9. Dispositif pour administrer un produit fluide selon l'une quelconque des revendications 7 ou 8, **caractérisé en ce que** le dispositif de blocage (5) comprend un moyen de verrouillage qui, en position de blocage, verrouille un organe d'entraînement (3) contre l'avance.

10. Dispositif pour administrer un produit fluide, comprenant :
- un boîtier (1) pour recevoir un dispositif d'administration ;
- un élément d'actionnement (6) pour actionner le dispositif d'administration ;
- un dispositif de réception (2) pour recevoir le produit fluide ;
- un dispositif de blocage (5, 23) pour bloquer l'élément d'actionnement (6),
- le dispositif de réception (2) étant monté de manière rotative par rapport au boîtier (1),
- le dispositif de blocage (5, 23) étant déplaçable depuis une position de blocage jusqu'à une position de déblocage en faisant tourner le dispositif de réception (2) par rapport au boîtier (1),
un dispositif d'arrêt étant prévu qui arrête le dispositif de réception par rapport au boîtier lorsque le dispositif de blocage est dans sa position de déblocage, de sorte que le dispositif de réception ne peut être tourné que dans une seule direction de rotation, afin de déplacer le dispositif de blocage (5, 23) depuis la position de blocage jusqu'à la position de déblocage, et ne peut pas être tourné encore en direction opposée dans la position de déblocage,
**caractérisé en ce que**
le dispositif de réception (2) reçoit une carpule à deux chambres (7), la carpule à deux chambres (7) pouvant être mélangée en faisant tourner le dispositif de réception (2).

11. Dispositif pour administrer un produit fluide selon la revendication 10, **caractérisé en ce que** la position de déblocage du dispositif de blocage (5, 23) correspond à une position de la carpule à deux chambres (7) dans laquelle la carpule à deux chambres (7) est mélangée.
